Europäisches Patentamt

European Patent Office

Office européen des brevets

(11) Publication number: 0 273 468
A2

# EUROPEAN PATENT APPLICATION

(21) Application number: 87202206.6

(22) Date of filing: 22.12.82

(51) Int. Cl.⁴: A61B 17/10

(30) Priority: 22.12.81 AU 2053/81
28.06.82 AU 4621/82

(43) Date of publication of application:
06.07.88 Bulletin 88/27

(60) Publication number of the earlier application in
accordance with Art.76 EPC: 0 096 694

(84) Designated Contracting States:
AT CH DE FR GB LI SE

(71) Applicant: HOSPITAL Products Limited
c/o Deloitte Haskins and Sells Chartered
Accountants 255 George Street
Sydney New South Wales 2000(AU)

(72) Inventor: Freund, Hans Ludwig
208 Woniora Road
South Hurstville, NSW 2221(AU)
Inventor: Kerr, Gavin
14 Kathleen Avenue
Castle Hill, NSW 2154(AU)

(74) Representative: Richards, David John et al
Page, White & Farrer 5, Plough Place New
Fetter Lane
London EC4A 1HY(GB)

(54) Surgical stapling instrument.

(57) A surgical stapler of the type in which staples are ejected from a staple cartridge to be formed against an anvil, the cartridge being held in a cartridge holder (122) between the jaws (55), (56) of an interchangeable jaw member (54). The jaw member (54) is interchangeably attached by a locking slide (53) to a housing (50) having an operating lever (58). The operating lever (58) is operable in a first movement to cause the cartridge holder (122) to move towards the anvil placing the cartridge into a staple firing position and is operated in a second movement to cause the staple to be ejected from the cartridge.

Fig. 1

## SURGICAL STAPLING INSTRUMENT

This invention relates to surgical stapling instruments of the type in which staples are ejected from a staple cartridge to be formed against an anvil surface mounted in juxtaposition with the cartridge, the staple cartridge being movable relative to the anvil between retracted and staple firing positions.

Prior art instruments of this type are well known, and are exemplified by the instrument described in U.S. Patent 3,490,675. The prior art instruments comprise, in addition to jaws or other means of supporting the anvil surface and a cartridge holder, an elongated housing containing the drive mechanism, an operating handle and means for the movement of the cartridge holder.

A principal object of the present invention as applied to instruments of the type in which the anvil surface and the staple cartridge are mounted between opposed jaws, is to enable interchange of the jaws, so that jaws and cartridge/anvil assemblies of differing standard sizes (e.g. 90 mm, 55 mm, 30 mm) may be employed with the same actuating unit, thereby effecting a substantial cost saving in the hands of consumers.

In the prior art instruments, the function of tissue proximation, by movement of the position of the staple cartridge from its retracted position to the staple firing position, and the function of staple firing have been carried out by separate operations. Tissue proximation is achieved by means of a screw-operated device, and firing by means of the actuating lever. In the instrument described herein, both functions of tissue proximation and staple firing are carried out by successive movements of the operating lever.

In broad form, the invention comprises a surgical stapling instrument of the type in which staples are ejected from a staple cartridge to be formed against an anvil surface mounted in juxtaposition with said cartridge, said cartridge being movable between a staple firing position at a predetermined distance from said anvil surface and a retracted position at a greater distance from said anvil surface, said instrument comprising a cartridge holder movable to control the position of said cartridge and a staple driving member movable towards said cartridge to cause ejection of staples therefrom, said anvil surface being provided on a forward portion of a stapling assembly comprising said cartridge holder and said driving member, characterised in that said cartridge holder and said driving member are provided with respective driving portions extending rearwardly therefrom, each of said driving portions having formations adjacent the rearward end thereof for detachable engagement of said portions respectively with cartridge pusher means and driving member actuating means provided in a stapling instrument housing, said stapling assembly having means for the detachable mounting thereof on said instrument housing.

## BRIEF DESCRIPTION OF THE DRAWINGS

In the accompanying drawings:

Fig. 1 shows a stapling instrument according to the present invention in side elevation;

Fig. 2 is a partly sectioned side elevation of the instrument with one housing half removed to show the operating mechanism;

Fig. 3 is a sectional plan taken on the line 3-3 of Fig. 2 with the mechanism of the instrument in its fired condition;

Fig. 4 is a side elevation of an instrument housing half showing portion of the instrument operating mechanism;

Fig. 5 is a section taken on the lines 5-5 of Fig. 4;

Fig. 6 is a cross-section taken on the line 6-6 of Fig. 4;

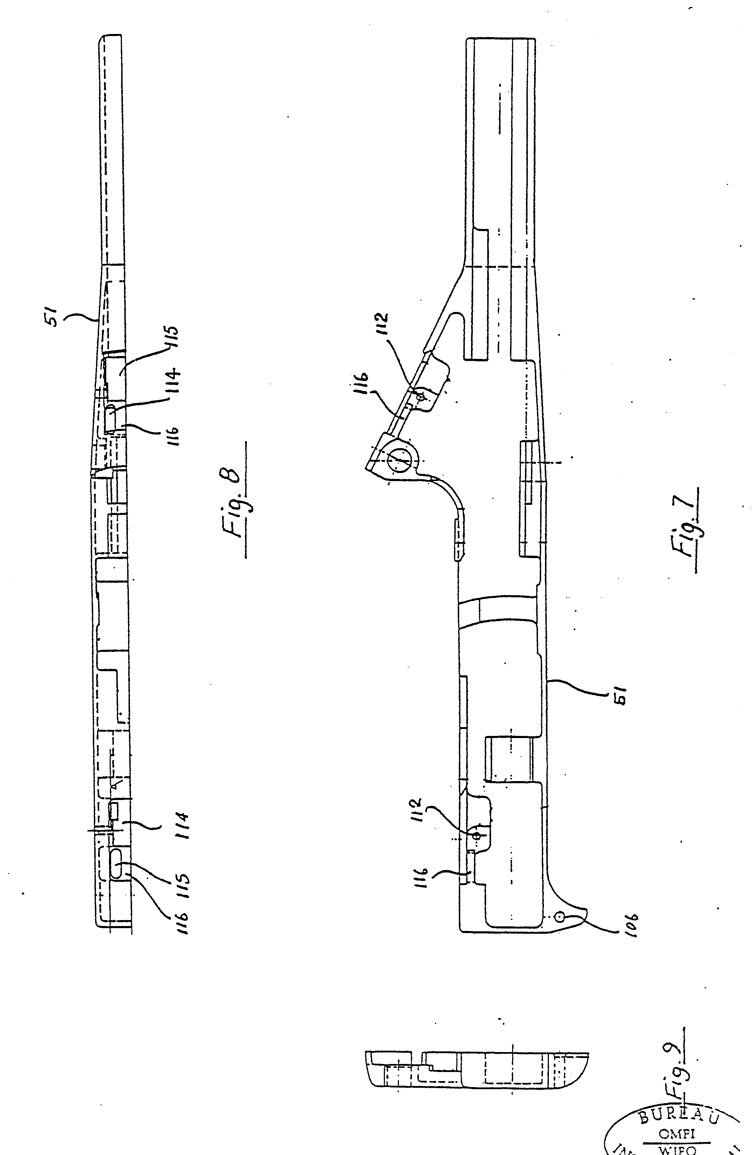

Fig. 7 is a side elevation of the other housing half of the instrument;

Fig. 8 is a plan view of the housing half illustrated in Fig. 7;

Fig. 9 is an end elevation of the housing half shown in Fig. 7;

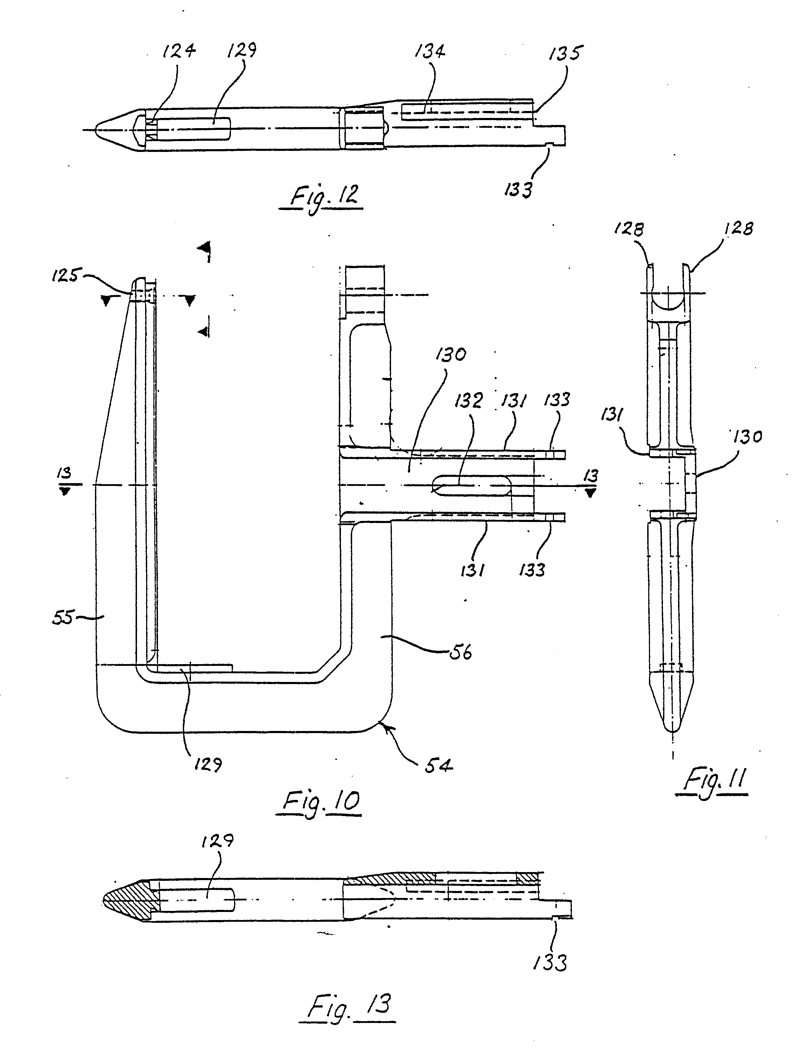

Fig. 10 is a side elevation of a jaw suitable for use with the instrument of Figs. 1 to 9;

Fig. 11 is an end elevation of the jaw illustrated in Fig. 10;

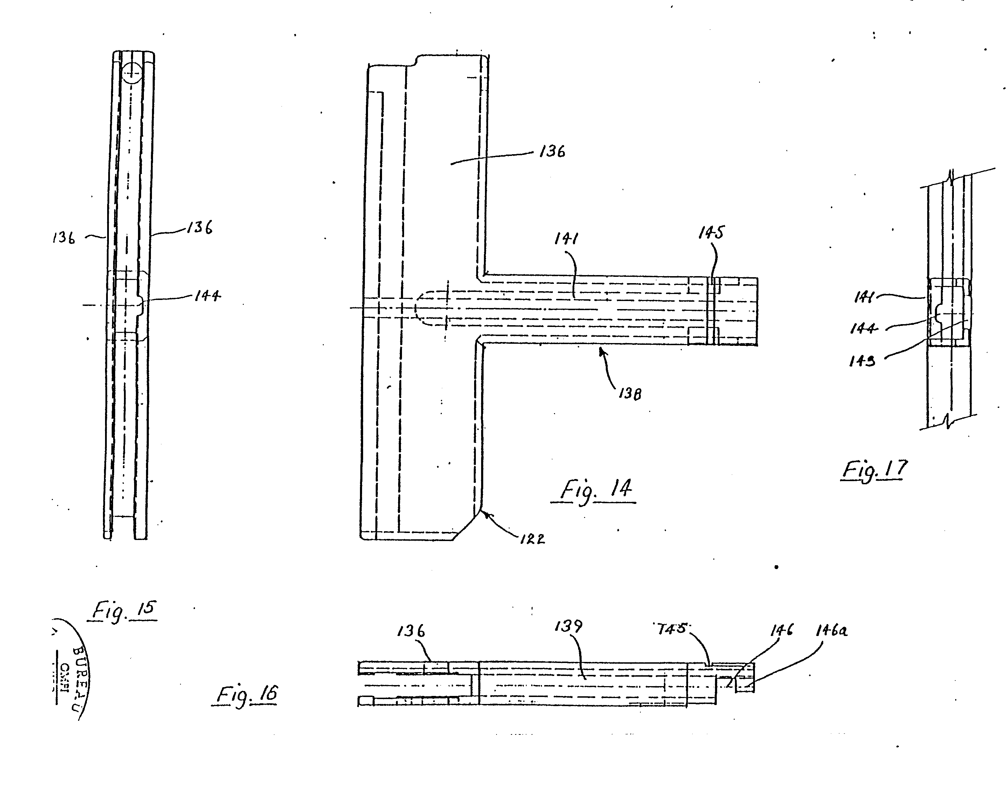

Fig. 12 is a plan view of the jaw illustrated in Fig. 10;

Fig. 13 is a section taken on the line 13-13 of Fig. 10;

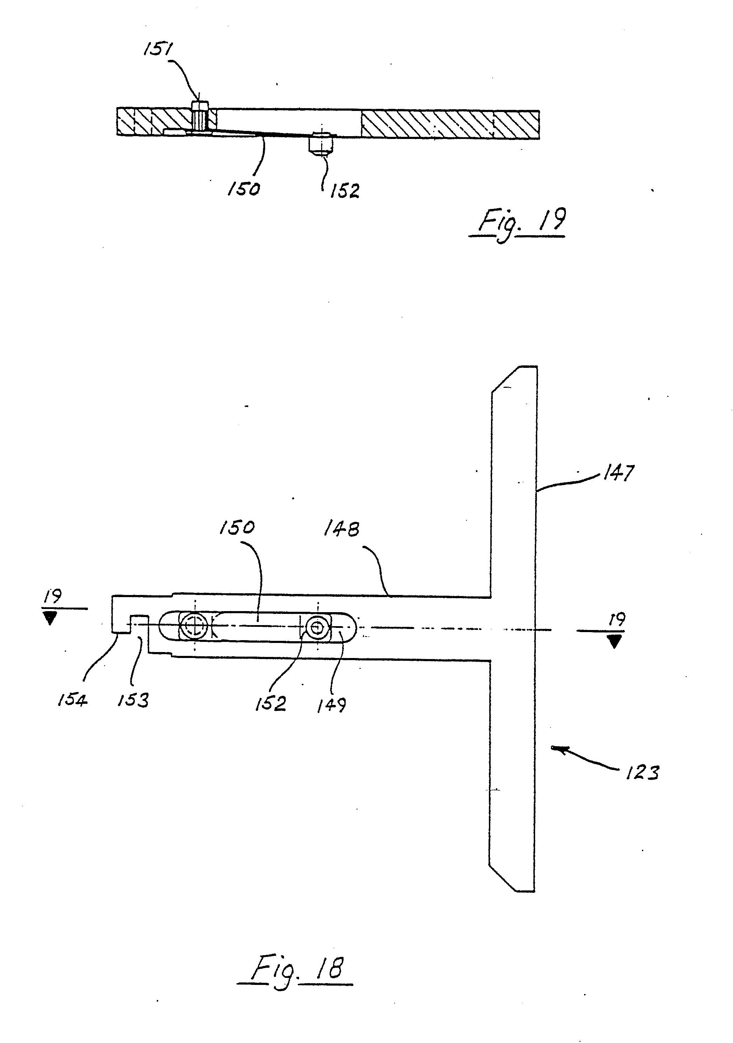

Fig. 14 is a side elevation of a cartridge holder suitable for use in the illustrated instrument;

Fig. 15 is an end elevation of the cartridge holder of Fig. 14;

Fig. 16 is a bottom plan view of the cartridge holder;

Fig. 17 is a fragmentary end elevation of the cartridge holder illustrated in Fig. 14;

Fig. 18 is a side elevation of a T-bar;

Fig. 19 is a section taken on the line 19-19 of Fig. 18;

Fig. 20 is a side elevation of a locking slide;

Fig. 21 is a plan view of the locking slide of Fig. 20;

Fig. 22 is an end elevation of the locking slide;

Fig. 23 is a side elevation of the head assembly of the illustrated instrument;

Fig. 24 is an end elevation of the head assembly;

Fig. 25 is a section taken on the line 25-25 of Fig. 23;

Fig. 26 is a section taken on the line 26-26 of Fig. 23;

Fig. 27 is a side elevation of a T-bar extension;

Fig. 28 is a plan view of the T-bar extension;

Fig. 29 is a section taken on the line 29-29 of Fig. 28;

Fig. 30 is a side elevation of a pusher;

Fig. 31 is a plan view of the pusher;

Fig. 32 is an end elevation of the pusher;

Fig. 33 is a side elevation of a latch;

Fig. 34 is an end elevation of the latch;

Fig. 35 is a schematic elevation of the instrument mechanism in a first actuated position; and

Fig. 36 is a schematic elevation of the mechanism in a second actuated position.

## BEST MODE FOR CARRYING THE INVENTION

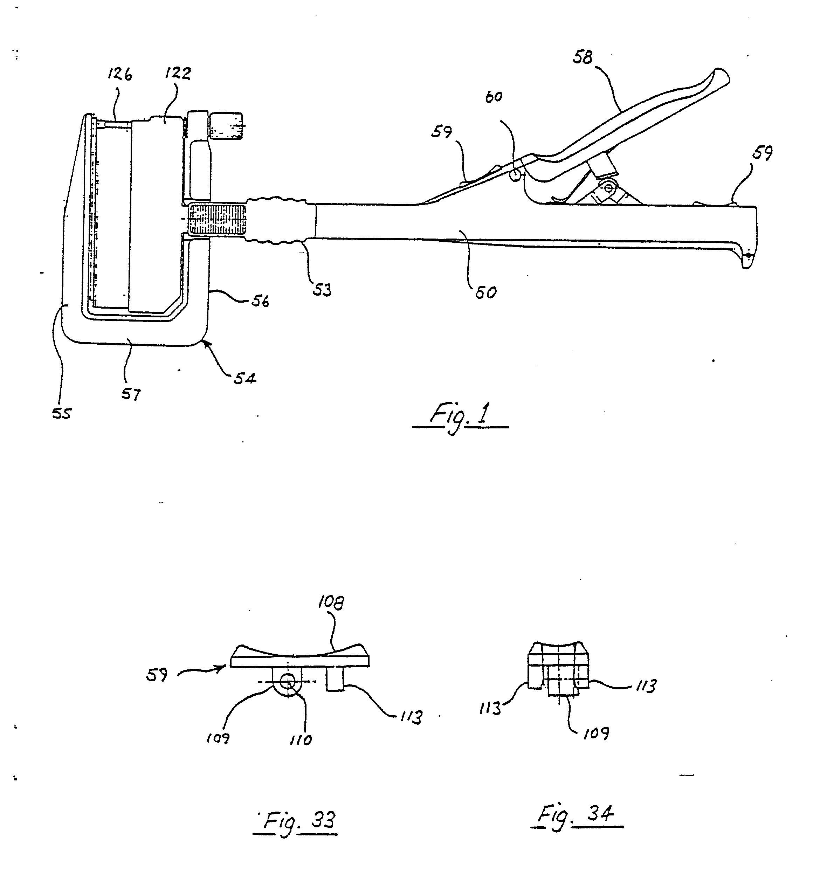

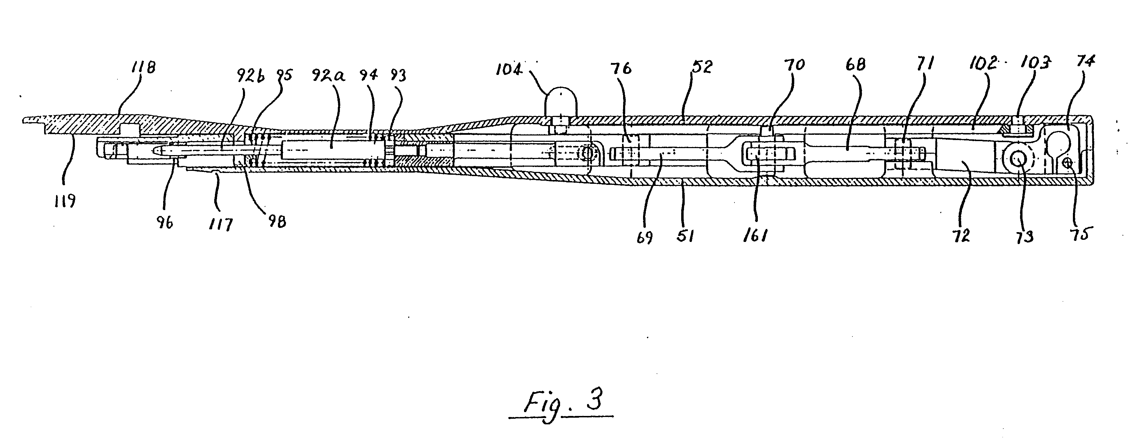

The illustrated instrument shown generally in Fig. 1 comprises a housing 50 made up, as shown in Fig. 3, of a left-hand housing half 51 and a right-hand housing half 52. Detachably mounted at the forward end of the instrument housing 50, with the aid of a locking slide 53, is a jaw member 54 comprising respective forward and rear jaws 55 and 56 and a bight portion 57. Pivotally mounted on the rear portion of the housing 50 is an operating lever 58.

The housing halves 51 and 52 are held together at the rear and at the central portions of the instrument, by means of pivoting latches 59 which will be described in detail below. At the forward end of the instrument housing 50, the halves 51 and 52 are held together by the locking slide 53 as described hereinafter.

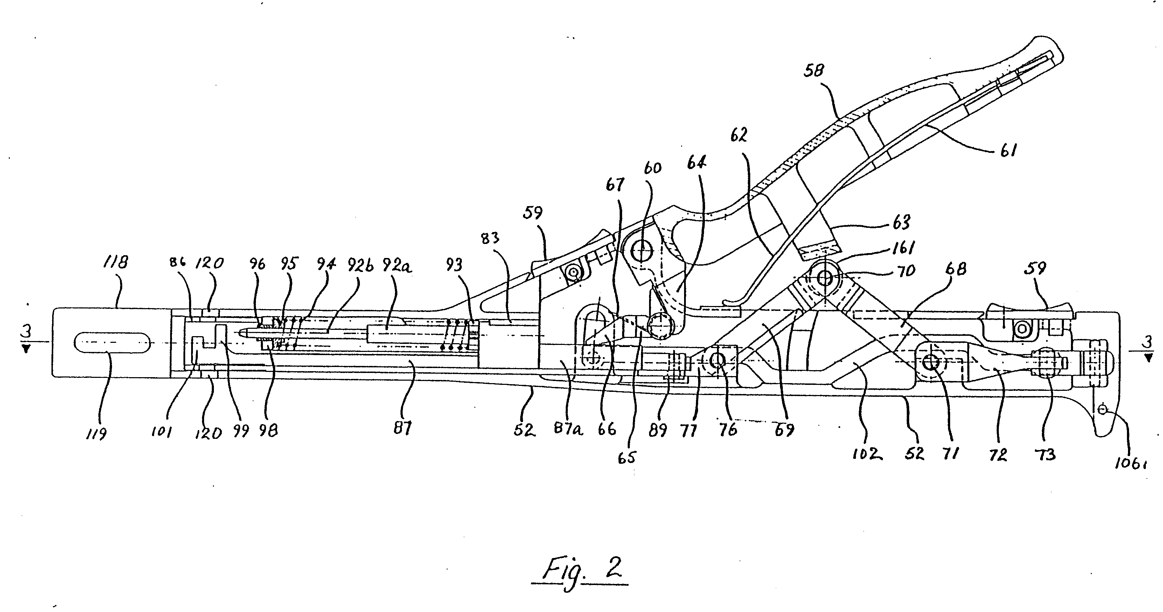

The operating lever 58 is mounted on a pivot pin 60 the ends of which are located in the respective housing halves 51 and 52. A leaf spring 61, the free end 62 of which bears on the upper surface of the housing 50, biases the lever 58 to its open position as illustrated in Fig. 2. Extending downwardly from the central portion of the lever 58 is a toggle driving member 63, while depending from the forward end of the lever 58 is a member 64 on the lower end of which is pivotally mounted a pawl 65, having a downwardly and forwardly extending nose portion 66. The pawl 65 is biased downwardly by means of a spring 67.

Lying within the housing 50 is a toggle comprising a rear toggle link 68 and a forward toggle link 69, these links being interconnected by a toggle pin 70. This toggle pin carries a roller 161 for engagement by the toggle driving member 63 as described below. The rear toggle link 68 is connected by means of a pin 71 with a member 72 the rear end of which is connected, by means of a pin 73, with a pressure plate 74 fixed to the housing half 52 by means of a pin 75. The function of the plate 74 will be described below.

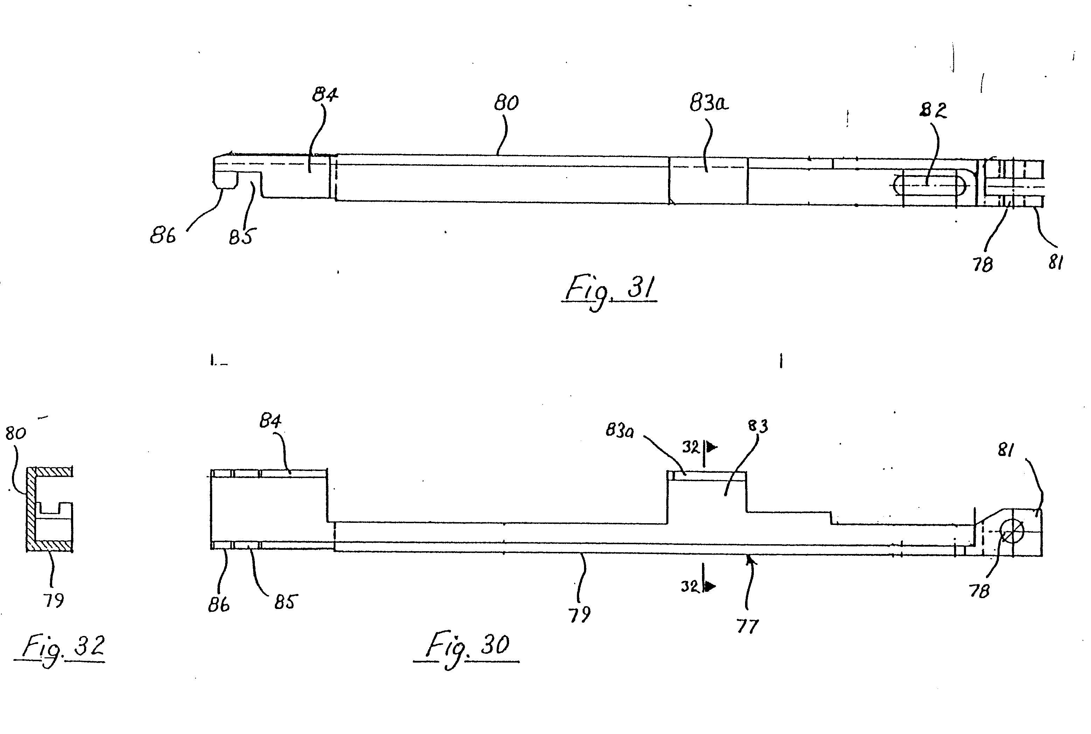

At its forward end, the forward toggle link 69 is connected by means of a pivot pin 76 with the rearward end of a pusher member 77, the pin 76 passing through apertures 78 in the rear end of the pusher 77, which is shown in detail in Figs. 30 to 32.

The pusher 77 is shaped and dimensioned for longitudinal sliding movement within the housing 50, and comprises a base 79 and a side wall 80. The apertures 78 are provided respectively in the side wall 80 and in a web 81, and an elongated slot 82 is provided in the base 79 of the pusher 77 for a purpose described below.

In the middle region of the pusher 77, the side wall 80 is extended upwardly by web 83 and a flange 83a extends inwardly from this extended portion of the side wall, for a purpose to be described below. Similarly, at the forward end of the pusher 77 the side wall 80 is again extended upwardly, and a flange 84 extends laterally from this portion. The flange 84 and the underlying portion of the base 79 are similarly formed with slots 85 and inwardly directed lugs 86.

Mounted within the pusher 77 for longitudinals sliding movement relative thereto, is a T-bar extension 87, shown in detail in Figs. 27 to 29. At the rear end of the T-bar extension 87 a vertical hole 88 is provided, for the reception of a pin 89 (Fig. 2). This pin has an enlarged head and passes through the slot 82 in the base 79 of pusher 77, the head of the pin lying below the base 79, this pin thereby functioning to retain the T-bar extension 87 in assembly with the pusher 77, the slot 82 allowing for the necessary relative longitudinal movement of the T-bar extension 87.

Forward of the pin 89, the T-bar extension 87 is provided with a portion 87a of increased height, to form a rearwardly facing shoulder 87b, the purpose of which will be explained below.

The T-bar extension 87 is also provided near the middle thereof, with a raised portion containing a drilled hole 90. This raised portion is dimensioned for a neat fit beneath the flange 83 of the pusher 77, and is provided at its forward end with a shoulder 91 and 91a which bear against the forward edges of the web 83 and the flange 83a to limit the rearward relative movement of the T-bar extension 87 with respect to the pusher 77.

The hole 90 is tapped to receive the threaded rear end of a stop pin 92 comprising a body 92a and a forwardly extending spring guide portion 92b. By virtue of this screwed connection with the T-bar extension 87, the stop pin 92 is longitudinally adjustable, and is locked in its desired position by means of a lock nut 93 which bears against the surface of the T-bar extension 87 surrounding the threaded hole 90. Surrounding the stop pin 92 is a helical compression return spring 94, the rearward end of which bears against the lock nut 93 and the forward end of which bears against a spring retainer 95 which is slidably mounted on the forward portion of the stop pin 92. To prevent the escape of the return spring 94 and the spring retainer 95 from the stop pin when the instrument is disassembled for cleaning, a retaining nut 96 is screwed on to the threaded forward end of the stop pin 92.

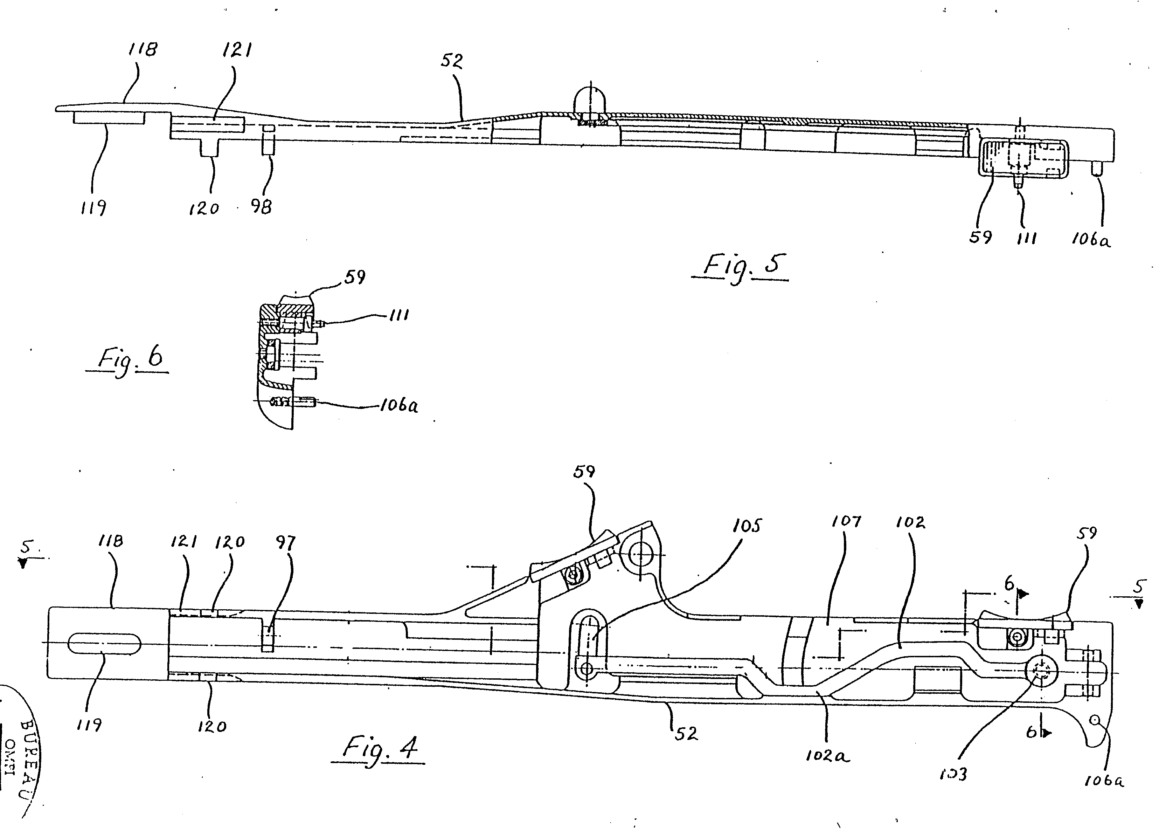

The forward end of the stop pin 92 passes through a slot 97 in a stop lug 98 (Figs. 4 and 5) which extends inwardly from the upper portion of the side wall of the right-hand housing half 52. The slot 97 is open at the left-hand extremity of the lug 98, so that the stop pin and spring assembly may be freed from the housing when the drive assembly is removed for cleaning.

At the forward end of the T-bar extension 87 an upwardly extending lug 99 is separated by a slot 100 from a further upwardly extending lug 101 at the extreme forward end of the T-bar extension.

Mounted within the rear portion of the housing 50 is a release lever 102. This lever, which is best seen in Figs. 3 and 4, is pivotally fixed at its rear end to the right-hand housing half 52 by means of a pivot 103. At the forward end of the lever 102, a release lever button 104 passes through an arcuate slot 105 in the right-hand housing half 52. In the central region of the release lever 102 there is provided a depressed portion 102a which lies below the right-hand end of the toggle pin 70, which is extended beyond the toggle members towards the right-hand housing half 52.

Apart from the latches 59 referred to above, engagement of the housing halves is guided by means of a locating pin 106a anchored in an aperture in the right hand housing half 52 and adapted to enter an aperture 106 in the other housing half. The upper wall of each housing half 51 and 52 is open at 107 to provide clearance for the toggle links 68 and 69 when that toggle is in its contracted condition as illustrated in Fig. 2.

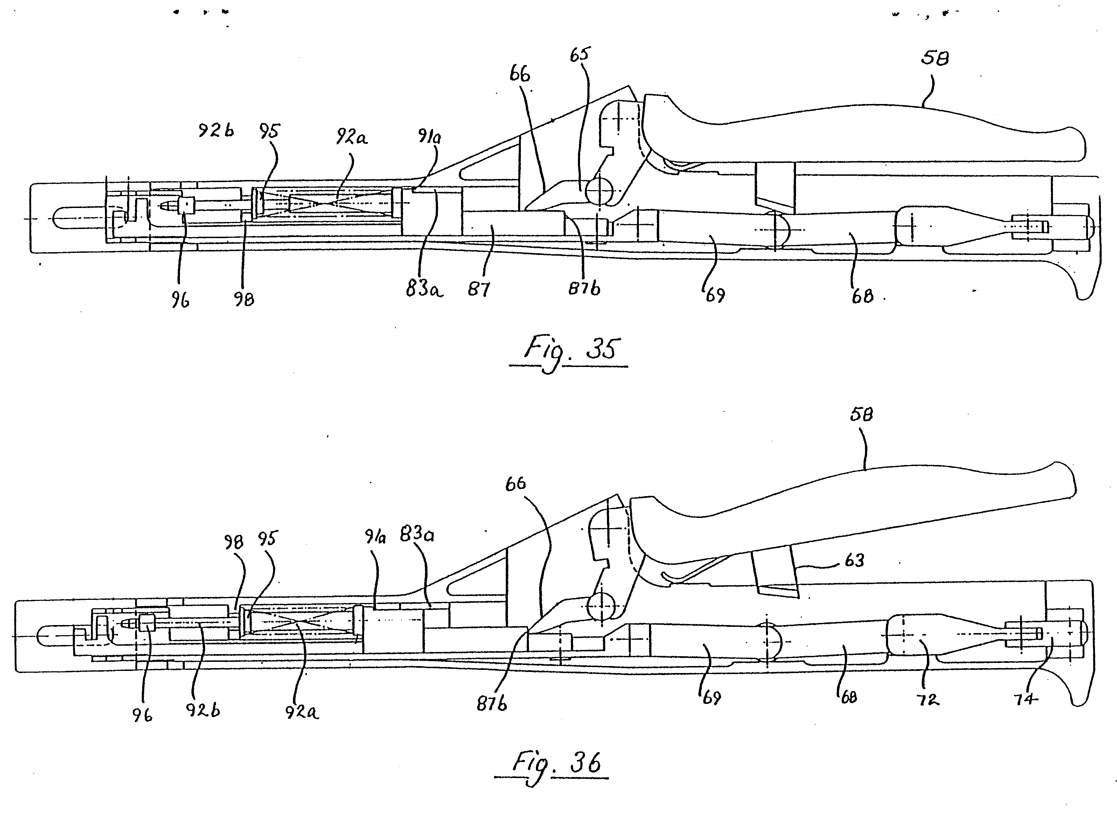

The latches 59 are illustrated in Figs. 33 and 34, and comprise a housing portion 108 the upper surface of which is concavely contoured for ease of operation with the thumb or finger, and depending donwwardly from the housing 108 there is provided a lug 109 which is provided with a drilled hole 110 for the reception of a pivot pin 111 (Fig. 6), one end of which is anchored in the right-hand housing half 52, and the other end of which is located in an aperture 112 in the left-hand housing half. Towards one end of the housing 108, the latch is provided with a pair of opposed downwardly extending lugs 113.

The upper wall of each housing half 52 and 53 is apertured at 114 to allow for rotation of the latch on its pivot pin 111, and rearward of this aperture 114 there is provided a slot 115 for the reception of one of the lugs 113. These apertures 115 are provided in flanges 116 located below the upper surface of the respective housing half, by a distance corresponding to the base thickness of the latch housing 108. As will be appreciated, when the housing halves are correctly position together, each latch may be rotated from its open position to a closed position in which the lugs 113 engage within the respective apertures 115 of the adjacent housing halves, locking the housing halves together. As will be seen in Fig. 34, the inner surfaces of the lugs 113 are tapered to facilitate this locking action.

Adjacent the forward end of the left-hand housing half 51 is a vertical groove 117. The forward end of the right-hand housing half extends beyond that of the left-hand housing half in the region indicated at 118, this region having no bottom or upper wall. On the inner surface of this portion 118, there is provided a longitudinally directed key 119. Rearwardly of the portion 118, from the upper and lower walls of the right-hand housing half 52, there are provided inwardly directed lugs 120, and in the region of these lugs, the outer surfaces of the upper and lower walls of the right-hand housing half 52 are relieved to form grooves 121. The lugs 120 are located so that their rear edge abuts the front edge of the left-hand housing half 51, and extends laterally to an extent equal to the width of the left-hand housing half 51 at its forward end. The purpose of the key 119 and the grooves 121 is to enable locking assembly of the housing of the instrument with the head assembly, which will now be described.

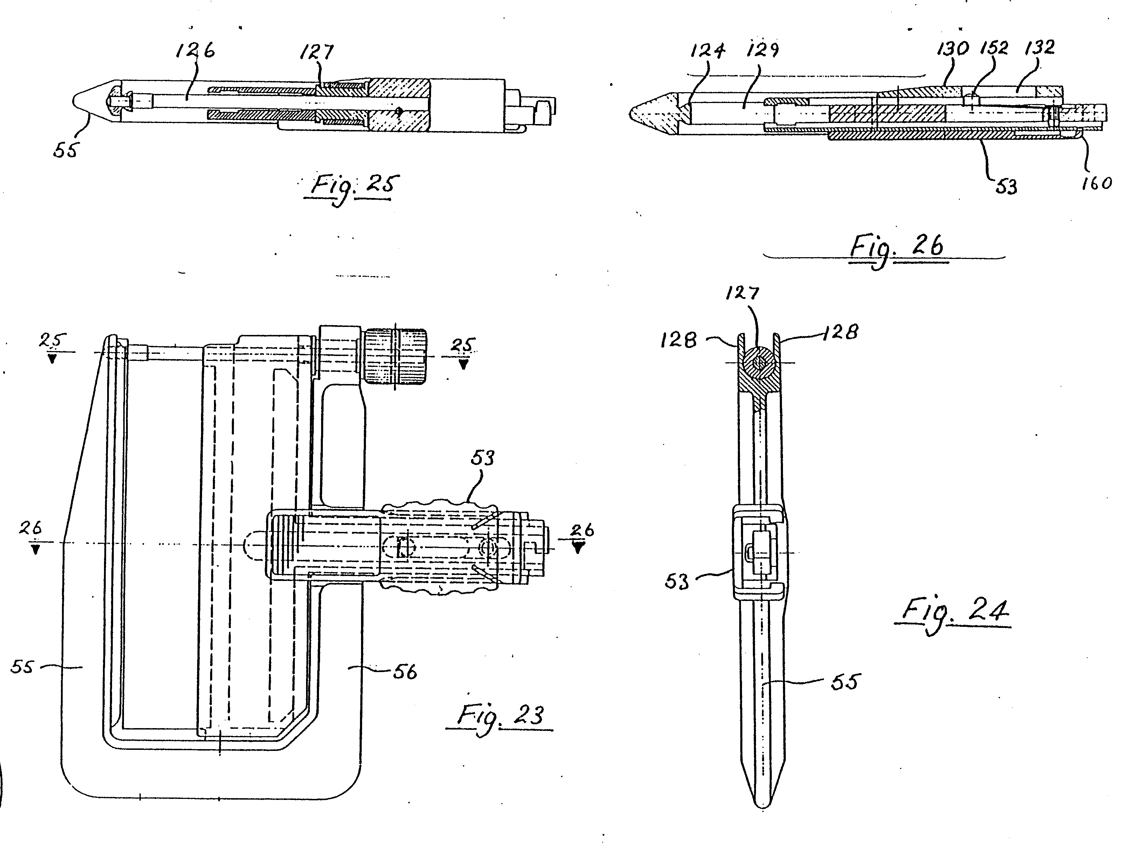

The head assembly of the instrument basically comprises the jaw member 54, a cartridge holder 122 and a T-bar 123. The T-bar is illustrated in Figs. 18 and 19 in a reversed position relative to the remainder of the components illustrated, in order that its locking features to be described below, may best be shown.

The jaw sections of the jaw member 54, comprising the forward jaw 55, rear jaw 56 and bight portion 57 have already been described. As in the prior art, the inner face of the forward jaw 55 is provided with an anvil receiving formation 124. An aperture 125 in the upper end of the forward jaw 55 receives the forward end of a tie pin 126, the rearward portion of which is passed through a cylindrical insert 127 which is held between flanges 128 which stand upwardly at the upper end of the rear jaw 56.

A guide rail formation 129 is provided in the forward region of the upper surface of the bight portion 57, for the purpose of guiding and laterally locating the bottom of the cartridge holder 122.

The upper and lower portions of the rear jaw 56 are joined by a web 130 provided with upper and lower flanges 131. The web 130 and the flanges 131 extend rearwardly from the jaw 56, and the web 130 is provided with a longitudinally orientated slot 132, this slot being dimensioned neatly to receive the key 119 provided on the forward portion 118 of the housing portion 52. The flanges 131 extend, in part, rearwardly for a short distance beyond the web 130, and vertically aligned slots 133 are provided at the left-hand side of the extending portions of these flanges 131. The upper and lower surfaces of the flanges 131 are relieved to provide grooves 134 which open to the rear edge of the flanges 131, in the region 135 where the flanges terminate coextensively with the web 130. These grooves 134 are located so as to align with the grooves 121 in the forward end of the right-hand housing half 52, and the formations described are dimensioned such that when the jaw member 54 is engaged with the instrument housing, the key 119 enters the slot 132, the portion 135 abuts the forward end of the upper and lower walls of the right-hand housing half 52, and the rearmost edge of each flange 131 abuts the forward edge of the respective lug 120.

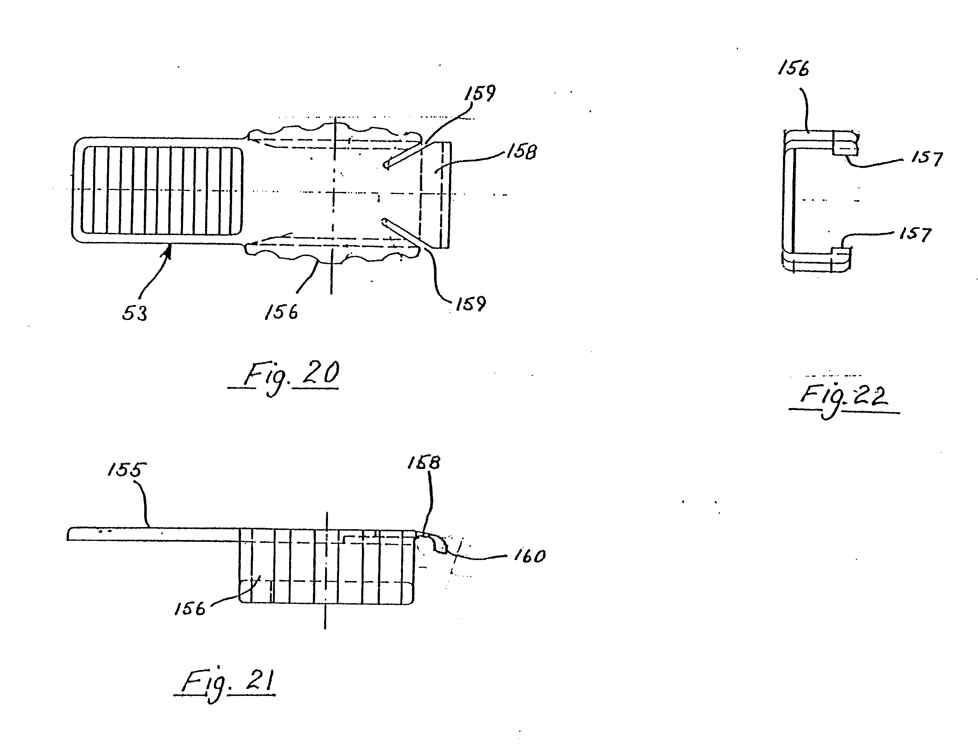

The cartridge holder 122 is of generally T-shape in side elevation, comprising a pair of head flanges 136 provided with opposed grooves 137 for the reception of a staple cartridge in the manner known in the prior art. Extending rearwardly from the head flanges 136 is a stem 138 comprising upper and lower walls 139 and 140 respectively, left-hand side wall 141 and a longitudinally slotted right-hand side wall 142. The slot 143 in the right-hand side wall 142 extends throughout the length of that side wall and continues forwardly through portion of the right-hand head flange 136. On the inner surface of the left-hand side wall 141 there is provided a groove 144 which runs for the entire length of this side wall and continues for the entire longitudinal extent of the left-hand head flange 136. On the outer surface of the left-hand side wall 141 forwardly of the rear thereof, there is provided a vertical groove 145, and the rear portion of the right-hand side wall is removed to allow the formation in the upper and lower walls 139 and 140, of a slot 146 and lug 146a (Fig. 16). These formations are dimensioned and located such that upon engagement of the head assembly with the instrument housing, the lugs 146 enter the slots 85 at the forward end of the pusher 77, and the lugs 86 at the forward end of the pusher 77 enter the slots 145.

As illustrated in Figs. 18 and 19, the T-bar 123 comprises a head 147 and a stem 148. The stem 148 is slotted at 149 and in this region a leaf spring 150 is provided, mounted at its rear end by means of a pin 151. The leaf spring 150 is biased outwardly of the slot 149 towards the right-hand side of the T-bar, and at its forward end there is mounted a locking pin 152. The locking pin 152, in the relaxed condition of the spring 150, stands proud of the right-hand side of the T-bar 123. The T-bar is, of course, mounted within the cartridge holder 122, with its head 147 between the head flanges 136 of the cartridge holder, and its stem 148 within the stem 138 of the cartridge holder. The pin 151 extends beyond the left-hand side of the T-bar and runs in the groove 144 in the left-hand side wall of the cartridge holder, while the locking pin 152 is free to lie within the slot 145 in the right-hand side wall 142 of the cartridge holder stem 138, and projecting outwardly beyond that side wall.

At the rearward end of the stem 148 there is provided a slot 153 and a downwardly directed lug 154, the lug 154 being located and dimensioned to engage with the slot 100 in the forward end of the T-bar extension 87 upon engagement of the head assembly with the instrument housing.

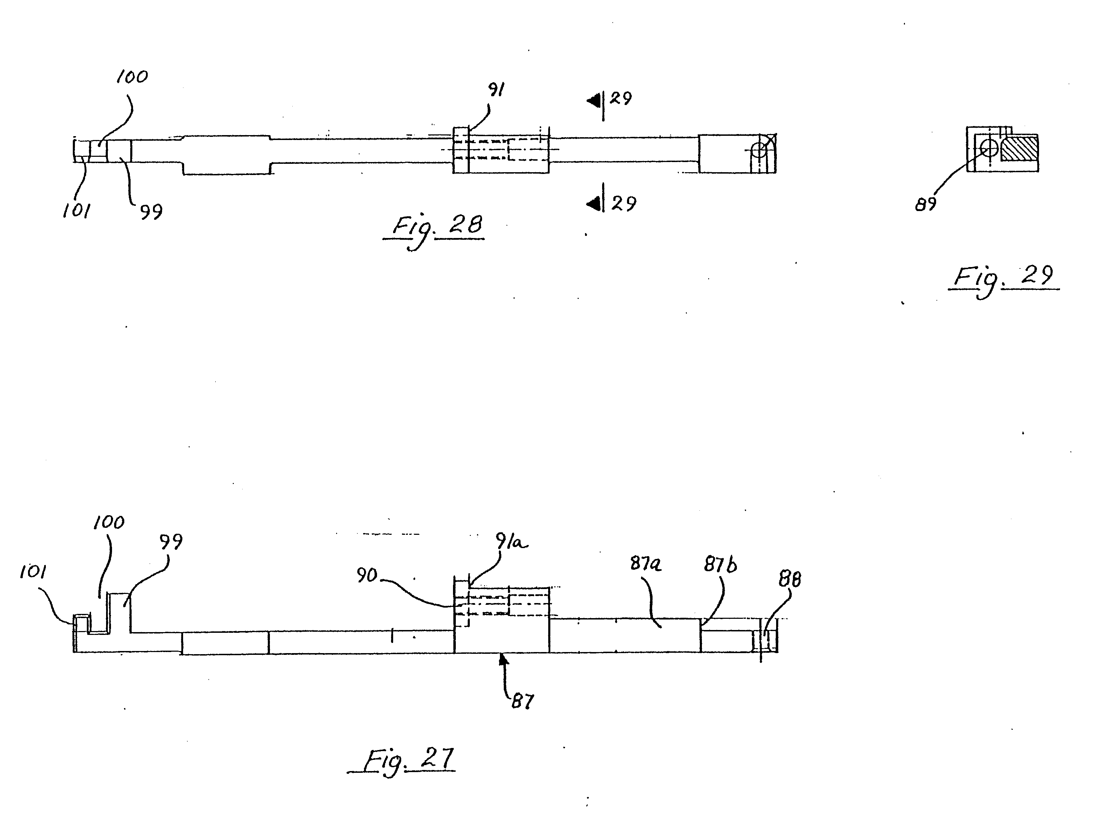

The head assembly is completed by the locking slide 53, which is illustrated in Figs. 20 to 22. The locking slide 53 comprises a web 155 and a pair of flanges 156. The outer surface of the flanges 156 is provided with vertical grooves to assist the operator's grip, and each flange 156 is provided with opposed inwardly directed flanges 157.

A resilient tongue 158 is formed at the rear end of the web 155, by means of diagonal slots 159, and the rear edge of the tongue 158 is provided with an inwardly directed detent 160.

Figs. 23 to 26 show the relationship between the components of the head assembly, in their assembled condition prior to engagement of the head assembly with the remainder of the instrument. The locking slide 53 embraces the stem 138 of the cartridge holder 122, with the flanges 157 engaged within the grooves 134, while the detent 160 is snapped into the groove 133. The locking pin 152 of the T-bar 123 extends through the slot 145 in the cartridge holder stem 138 to enter the slot 132 in the web 130, thereby restricting longitudinal movement of the T-bar in this condition.

The immediately apparent advantage of this arrangement by which the components of the head assembly are held in their correct relative positions is the facilitation of correct engagement of the head assembly with the remainder of the instrument. Other advantages flow however, in particular the instrument described enables the surgeon to position the head assembly in relation to the patient before attaching the housing and drive mechanism, and it will be appreciated that this offers advances in the surgical techniques and increases the utility of the instrument.

The head assembly is engaged with the instrument housing and actuating mechanism, with the T-bar 123 in its forwardmost position as defined by the engagement of the locking pin 152 with the forward end of the slot 132, by longitudinally aligning the housing and head assembly and moving the latter from the left such that the key 119 enters the slot 132. As this occurs the T-bar extension 87 will engage with the T-bar 123 and the pusher 77 will engage with the cartridge holder 122 as previously described, and the key 119 will press the T-bar locking pin 152 inwardly and out of the slot 132, freeing the T-bar for operative longitudinal movement. The operator now grips the locking slide 53 and moves it rearwardly, so that the flanges 157 move into the grooves 121 and the flanges 156 embrace the upper and lower walls of both housing parts 51 and 52, and the flanges 131 of the jaw member 54. The housing parts 51 and 52 are pressed together by the web 155.

The locking slide 53 is drawn on to the housing until the detent 160 engages the groove 117 in the left hand housing part 51. With the mounting of a cartridge and anvil in known manner, the instrument is then ready for use.

The operation of the instrument thus described is as follows, and reference should be made to Figs. 2, 23 and 24. In Fig. 2 the mechanism is shown in its initial condition. Tissue proximation is first achieved by depressing the operating lever 58 so that the toggle drive member 63 contacts the toggle centre roller 161, driving this downwardly and thereby driving the pusher 77 forward by virtue of its engagement with the forward toggle pin 76. The cartridge holder 122 is thus moved to the staple firing position.

The return spring 94 urges the T-bar extension rearwardly so that its shoulder 91 is urged against the pusher flange 83. The T-bar extension, and thus the T-bar, will therefore move forward with the pusher.

The mechanism will now be in the position shown in Fig. 35. During this movement, the pawl 66 also moves forwardly, but with no function.

When the operating lever 58 is released and allowed to return to its open position with the aid of the leaf spring 61, the pawl 66 will return to its rearmost position, and the pusher and T-bar extension will remain in their forward position due to the toggle 68 and 69 having been driven over-centre.

With the T-bar extension 87 thus moved forward, the returning pawl 66 will drop behind the T-bar extension shoulder 87b. On the second depression of the operating lever therefore, the pawl 66 will engage this shoulder and drive the T-bar extension 87, and thus the T-bar 123, forward, producing staple firing. This position of the mechanism is illustrated in Fig. 36.

Forward motion of the T-bar extension will be arrested when the shoulder formed by the forward end of the stop pin housing 92a is driven up against the spring retainer 95, which bears against the stop lug 98. As mentioned above, this limit of travel is adjustable by virtue of the screwed mounting of the stop pin 92.

As will no doubt be appreciated, the function of the pressure plate 74 is to absorb the effects of excessive tissue thickness or other variations which may effect the stapling force and staple formation. This member acts as a compression spring, allowing the entire drive mechanism to move backwards under excessive stapling force, and thereby increasing the effective tissue gap.

Other types of residual devices may be substituted for the pressure plate 74, such as constant-rate springs.

Thus the steps of tissue proximation and staple firing are carried out by successive movements of the one operating member, considerably simplifying the operation of the instrument. This action, combined with the disposition of the operating lever 58, enables true one-handed operation to be achieved.

The sequence of operations is completed by the operator moving upwardly (as viewed in the drawings) the release button 104. This raises the release lever 102 as it pivots about the pin 103, and the portion 102a contacts the extended portion of the toggle pin 70, forcing the toggle through its centre of motion, whereupon the pressure of the return spring on the T-bar extension 87 and the pusher 77 will return the toggle to its initial position.

The drive mechanism thus described has a further advantage which arises from the fact that the pusher, T-bar extension, toggle links, and pivot link 72 will remain interconnected when lifted away from the right-hand housing half 52 for cleaning, the pivot link 72 enabling this movement while retaining the connection of these components to the housing half. Thus the mechanism may be cleaned between actuations with the minimum delay in re-assembly, such re-assembly requiring no special techniques and no tools. It will be appreciated that the use of latches 59 to retain the housing halves together, in cooperation with the locking slide 53, also greatly simplifies the partial disassembly of the instrument for cleaning.

The ease with which head assemblies may be interchanged in the instrument of the present invention enables such head assemblies, comprising jaws, cartridge holder, T-bar and cartridge to be disposable, should this be desired.

It will be appreciated that while one embodiment of the invention has been described in detail, the purpose of this description is solely to show the best manner of performance of the principles of the invention presently known, and it will be understood that many variations and alternative mechanical arrangements by which these principles may be realised are possible.

## Claims

1. A surgical stapling instrument of the type in which staples are ejected from a staple cartridge to be formed against an anvil surface mounted in juxtaposition with said cartridge, said cartridge being movable between a staple firing position at a predetermined distance from said anvil surface and a retracted position at a greater distance from said anvil surface, said instrument comprising a cartridge holder movable to control the position of said cartridge and a staple driving member movable towards said cartridge to cause ejection of staples therefrom, said anvil surface being provided on a forward portion of a stapling assembly comprising said cartridge holder and said driving member, characterised in that said cartridge holder and said driving member have respective driving portions extending rearwardly therefrom, each of said driving portions having formations on the rearward end thereof enabling the detachable engagement of said portions respectively with cartridge pusher means and driving member actuating means provided in a stapling instrument housing, said stapling assembly having means for the detachable mounting thereof to said instrument housing.

2. A stapling instrument according to Claim 1 further characterised in that said instrument comprises locking means slideable longitudinally of the instrument between a locking position and a release position, said locking means engaging said stapling assembly and said housing in said locking position to retain said assembly on said housing.

3. A stapling instrument according to Claim 2 further characterised in that said housing consists of two(2) longitudinally extending halves, said locking means embracing the forward ends of each body half when in said locking position.

4. A stapling instrument according to Claim 3 further characterised by the provision of at least one locking lever mounted on one of said body halves for movement between an open and a closed position, means on said locking lever engaging the respective body halves to hold said halves together when said locking lever is in its locking position.

126   122

58

60

59

59

59

56

53

60

55   57   54

_Fig. 1_

108

59   →

113

113   113

109   110   109

_Fig. 33_

_Fig. 34_

_Fig. 2_

0 273 468

_Fig. 3_

0 273 468

PCT/AU82/00217

118  121                    52

119    120   98

_Fig. 5_

59

_Fig. 6_          111

106a

59

118  121  120  97          105      107   102          59

5

119

120

6

_Fig. 4_          52   102a

103          106a

6

5

0 273 468

PCT/AU82/00217

51

116 115   114

116   114  115

*Fig. 8*

116

112

116

112

106

51

*Fig. 9*

*Fig. 7*

_Fig. 12_

_Fig. 10_

_Fig. 11_

_Fig. 13_

0 273 468

136

136

136

141

145

144

138

122

*Fig. 14*

141

144

145

*Fig. 17*

*Fig. 15*

*Fig. 16*

136

139

145

146

146a

*151*

*150*    *152*

$$\underline{Fig.\ 19}$$

*147*

*150*    *148*

*19*    *19*

*154*    *153*    *152*    *149*

*123*

$$\underline{Fig.\ 18}$$

0 273 468

159

158

53

156

159

_Fig. 20_

156

157

157

_Fig. 22_

155

158

160

156

_Fig. 21_

Fig. 26

Fig. 24

Fig. 25

Fig. 23

WO 83/02247

100

91

◄|29

◄|29

*Fig. 28*

101    99

89

*Fig. 29*

0 273 468

100    99

101    90    91a    87a    87b    88

87

*Fig. 27*

PCT/AU82/00217

0 273 468

PCT/AU82/00217

_Fig. 31_

_Fig. 32_

_Fig. 30_

WO 83/02247

0 273 468

PCT/AU82/00217

*Fig. 35*

*Fig. 36*